# EUROPEAN PATENT APPLICATION

(11) **EP 4 047 345 A1**
(43) Date of publication of application: **24.08.2022**
(21) Application number: 20883616.3
(22) Date of filing: 20.07.2020
(51) Int. Cl.: G01N 15/14, G01N 37/00, C12M 1/00, B01J 19/00, C12N 1/02

(54) **MINUTE PARTICLE COLLECTION METHOD, MICROCHIP FOR ALIQUOTING MINUTE PARTICLES, MINUTE PARTICLE COLLECTION DEVICE, PRODUCTION METHOD FOR EMULSION, AND EMULSION**

(30) Priority: 31.10.2019 JP 2019198494
(71) Applicant: Sony Group Corporation, Tokyo 108-0075 (JP)
(72) Inventor: MATSUMOTO, Masahiro, Tokyo 108-0075 (JP); ITO, Tatsumi, Tokyo 108-0075 (JP)
(74) Representative: D Young & Co LLP
(86) International application number: PCT/JP2020/027986
(87) International publication number: WO 2021/084814

(57) **Abstract**

Provided is a new method for more efficiently generating emulsion particles each containing one fine particle.

The present technology provides a method of collecting fine particles, in which in a fine particle sorting mechanism having a channel structure including a main channel through which the fine particles flow, a collection channel into which particles to be collected are collected from among the fine particles, a connection channel that connects the main channel and the collection channel, and a liquid supply channel connected to the connection channel so as to supply a liquid, the method includes: a flow step of causing a first liquid containing the fine particles to flow through the main channel; a determination step of determining whether or not the fine particles flowing through the main channel are the particles to be collected; and a collection step of collecting the particles to be collected into the collection channel, and, in the collection step, the particles to be collected are collected into a second liquid that is immiscible with the first liquid in the collection channel while being contained in the first liquid.

## Description

### TECHNICAL FIELD

The present technology relates to a method of collecting fine particles, a microchip for sorting fine particles, a device for collecting fine particles, a method of producing an emulsion, and an emulsion. More specifically, the present technology relates to a method of collecting fine particles, a microchip for sorting fine particles, and a device for collecting fine particles, which are for collecting fine particles into an emulsion, a method of producing an emulsion including steps performed in the collection method, and an emulsion.

### BACKGROUND ART

In order to perform single cell analysis, the use of an emulsion in which each emulsion particle contains one cell has been studied. Several technologies for forming such an emulsion have been developed so far.

For example, Non-Patent Document 1 cited below discloses a method of randomly capturing particles in an emulsion. In this method, a solution containing cells is diluted to 100 cells/µl or less so that one emulsion contains one cell. In this method, efficiency of generating emulsion particles each containing one cell follows the Poisson distribution, and therefore a ratio of empty emulsion particles (emulsion particles containing no cell) is high, which is inefficient. In view of this, in order to increase a ratio of emulsion particles containing cells to empty emulsion particles, Non-Patent Document 1 cited below proposes sorting an emulsion by controlling particles or a fluid by, for example, an electric field, dielectrophoresis, local heating, or the like. Further, in order to increase a ratio of emulsion particles each containing one cell in a generated emulsion, for example, Non-Patent Document 1 cited below proposes using the plateau-Rayleigh instability induced by cells or using self-organization occurring when high-density suspended substances quickly pass through a microchannel.

### CITATION LIST

### NON-PATENT DOCUMENT

Non-Patent Document 1: Agata Rakszewska et al., One drop at a time: toward droplet microfluidics as a versatile tool for single-cell analysis, NPG Asia Materials (2014) 6, e133
Non-Patent Document 2: Linas Mazutis et al., Single-cell analysis and sorting using droplet-based microfluidic, Nat Protoc. 2013 May; 8 (5): 870-891

### SUMMARY OF THE INVENTION

### PROBLEMS TO BE SOLVED BY THE INVENTION

In order to perform single cell analysis using an emulsion, it is desirable to increase a ratio of emulsion particles each containing one cell in the emulsion. An object of the present technology is to provide a new method for more efficiently generating emulsion particles each containing one fine particle.

### SOLUTIONS TO PROBLEMS

The inventors of the present technology have found that the above problems can be solved by a specific method of collecting fine particles.

That is, the present technology provides a method of collecting fine particles, in which
in a fine particle sorting mechanism having a channel structure including
a main channel through which the fine particles flow,
a collection channel into which particles to be collected are collected from among the fine particles,
a connection channel that connects the main channel and the collection channel, and
a liquid supply channel connected to the connection channel so as to supply a liquid,
the method includes:
   a flow step of causing a first liquid containing the fine particles to flow through the main channel;
   a determination step of determining whether or not the fine particles flowing through the main channel are the particles to be collected; and
   a collection step of collecting the particles to be collected into the collection channel, and
   in the collection step, the particles to be collected are collected into a second liquid that is immiscible with the first liquid in the collection channel while being contained in the first liquid.

An emulsion containing the second liquid as a dispersion medium and the first liquid as a dispersoid can be formed in the collection channel by carrying out the method of collecting fine particles.

An emulsion can be formed by carrying out the method of collecting fine particles, and at least a part of droplets forming the emulsion can contain one of the particles to be collected.

The first liquid may be hydrophilic and the second liquid may be hydrophobic.

A kinematic viscosity of the second liquid can be 1/100 times to 100 times as much as the kinematic viscosity of the first liquid.

The flow step, the determination step, and the collection step can be performed while the second liquid is being supplied from the liquid supply channel to the connection channel.

The main channel may bifurcate into the connection channel and at least one waste channel through which a fine particle other than the particles to be collected flows, and
the liquid supply channel can supply the liquid to the connection channel. A valve for preventing the first liquid from entering the collection channel may be provided in the connection channel.

In the flow step, the fine particles can flow through the main channel substantially in a row toward the connection channel.

The fine particle sorting mechanism may have a channel structure in which a sample channel through which a liquid containing the fine particles flows and a sheath channel through which a liquid containing no fine particle flows are connected to the main channel at a joining portion so that the fine particles flow substantially in a row through the main channel after the joining portion, and
the channel structure can form a laminar flow containing the fine particles that flow substantially in a row.

In the determination step, the fine particles flowing through the main channel can be irradiated with light, and whether or not the fine particles are the particles to be collected can be determined on the basis of light generated by the irradiation.

In the collection step, the particles to be collected can be collected into the collection channel through the connection channel due to a pressure fluctuation in the collection channel.

The main channel, the connection channel, and the collection channel may be linearly arranged.

The fine particles may be cells or cell aggregation, and the first liquid may be a culture solution of the fine particles.

The fine particles may be cells, cell aggregation, or synthetic particles, and the fine particles may be destroyed after the collection step.

The fine particles collected into the collection channel may be subjected to further fine particle sorting processing.

A microchip for sorting the fine particles can have one or two channel structures.

Further, the present technology also provides a microchip for sorting fine particles, in which
the microchip has a channel structure including
a main channel through which the fine particles flow,
a collection channel into which particles to be collected are collected from among the fine particles,
a connection channel that connects the main channel and the collection channel, and
a liquid supply channel connected to the connection channel so as to supply a liquid,
the main channel has a determination region used for determining whether or not the fine particles flowing while being contained in a first liquid are the particles to be collected, and
the fine particles determined as the particles to be collected are collected into a second liquid that is immiscible with the first liquid in the collection channel while being contained in the first liquid.

Further, the present technology also provides a device for collecting fine particles including:
a microchip for sorting the fine particles having a channel structure including
a main channel through which the fine particles flow,
a collection channel into which particles to be collected are collected from among the fine particles,
a connection channel that connects the main channel and the collection channel, and
a liquid supply channel connected to the connection channel so as to supply a liquid;
a first liquid supply unit that supplies a first liquid containing the fine particles to the main channel;
a second liquid supply unit that supplies a second liquid that is immiscible with the first liquid to the liquid supply channel; and
a determination unit that determines whether or not the fine particles flowing through the main channel are the particles to be collected. The microchip for sorting fine particles may be detachable from the device for collecting fine particles.

Further, the present technology also provides a method of producing an emulsion containing emulsion particles containing fine particles, in which
in a fine particle sorting mechanism having a channel structure including
a main channel through which the fine particles flow,
a collection channel into which particles to be collected are collected from among the fine particles,
a connection channel that connects the main channel and the collection channel, and
a liquid supply channel connected to the connection channel so as to supply a liquid,
the method includes:
   a flow step of causing a first liquid containing the fine particles to flow through the main channel;
   a determination step of determining whether or not the fine particles flowing through the main channel are the particles to be collected; and
   a collection step of collecting the particles to be collected into the collection channel, and
   in the collection step, the particles to be collected are collected into a second liquid that is immiscible with the first liquid in the collection channel while being contained in the first liquid.

Further, the present technology also provides an emulsion containing emulsion particles containing fine particles, in which a ratio of the number of emulsion particles each containing one fine particle to the total number of emulsion particles is 70% or more.

### BRIEF DESCRIPTION OF DRAWINGS

Fig. 1 illustrates a configuration example of a microchip for sorting fine particles used in a method of collecting fine particles according to the present technology.
Fig. 2 shows an example of a flow of a method of collecting fine particles according to the present technology.
Fig. 3 is an enlarged view of an example of a particle sorting portion.
Fig. 4 is a block diagram of an example of a control unit.
Fig. 5 illustrates a configuration example of a microchip for sorting fine particles to which a container is connected.
Fig. 6A is an enlarged view of a connection channel portion.
Fig. 6B is an enlarged view of a connection channel portion.
Fig. 7A is an enlarged view of a connection channel portion.
Fig. 7B is an enlarged view of a connection channel portion.
Fig. 8A is photographs showing that an emulsion particle containing one fine particle is formed in a collection channel.
Fig. 8B is a photograph showing that emulsion particles having different sizes are formed under different driving conditions of a piezoelectric element.
Fig. 9 is a schematic diagram of an example of a microchip for sorting fine particles.
Fig. 10 is a schematic diagram of an example of a microchip for sorting fine particles.
Fig. 11 is a schematic diagram of an example of a microchip for sorting fine particles.

### MODE FOR CARRYING OUT THE INVENTION

Hereinafter, preferred modes for carrying out the present technology will be described. Note that embodiments described below are typical embodiments of the present technology, and the scope of the present technology is not limited to these embodiments. Note that the present technology will be described in the following order.
1. First embodiment (method of collecting fine particles)
   (1) Description of first embodiment
   (2) First example of first embodiment
      (2-1) Flow step
      (2-2) Determination step
      (2-3) Collection step
      (2-4) Other steps
         (2-4-1) Culture step
         (2-4-2) Destruction step
         (2-4-3) Detection step
         (2-4-4) Synthesis step
      (2-5) Fine particle sorting mechanism and fine particle
   (3) Other examples of channel structure
      (3-1) Channel structure in which main channel and waste channel are linearly arranged
      (3-2) Channel structure having plurality of collection channels
2. Second embodiment (microchip for sorting fine particles)
3. Third embodiment (device for collecting fine particles)
4. Fourth embodiment (method of producing emulsion)
5. Fifth embodiment (emulsion)

### 1. First embodiment (method of collecting fine particles)

### (1) Description of first embodiment

A method of collecting fine particles according to the present technology is performed by using a fine particle sorting mechanism having a channel structure including a main channel through which the fine particles flow, a collection channel into which particles to be collected are collected from among the fine particles, a connection channel that connects the main channel and the collection channel, and a liquid supply channel connected to the connection channel so as to supply a liquid. The method of collecting fine particles according to the present technology includes, in the fine particle sorting mechanism, a flow step of causing a first liquid containing the fine particles to flow through the main channel, a determination step of determining whether or not the fine particles flowing through the main channel are the particles to be collected, and a collection step of collecting the particles to be collected into the collection channel, and, in the collection step, the particles to be collected are collected into a second liquid that is immiscible with the first liquid in the collection channel while being contained in the first liquid.

According to the present technology, the particles to be collected are collected into the second liquid that is immiscible with the first liquid in the collection channel while being contained in the first liquid. Therefore, for example, emulsion particles containing the particles to be collected can be formed in the collection channel.

Further, in the present technology, the fine particles to be collected into the collection channel are particles determined as the particles to be collected in the determination step, and a collection operation is carried out at an appropriate timing. Furthermore, the collection operation is not performed when no fine particle flows or when fine particles that have been determined not as the particles to be collected in the determination step flows. Therefore, emulsion particles containing no fine particle or emulsion particles containing particles other than the particles to be collected are not formed in the collection channel. Therefore, there is an extremely high probability that each emulsion particle contains one particle to be collected. For example, the method according to the present technology makes it possible to generate emulsion particles each containing one fine particle (in particular, particle to be collected) at a success rate of, for example, 70% or more, particularly 80% or more, more particularly 90% or more, and further particularly 95% or more. In the present technology, the particles to be collected refer to fine particles determined to be collected in the determination step.

In single cell analysis using an emulsion, it is important to increase a content ratio of emulsion particles each containing one cell in the emulsion. It is particularly important to increase the content ratio in a case where single cell analysis is performed on a sample including a small number of cells.

However, for example, as disclosed in Non-Patent Document 2 cited above, it is considered that the probability of containing a certain number of cells in one emulsion particle follows the Poisson distribution. In a conventional emulsion forming technology, it is said that the probability of containing one cell in one emulsion particle is about 65% at the maximum.

Further, it is conceivable to increase the number of cells in a sample in order to increase the ratio. However, for example, the number of cells to be analyzed contained in a clinical sample is small in some cases. For example, the number of cells to be analyzed in one sample can be 10⁴ to 10⁵. Further, for example, in a case where rare cells such as circulating tumor cells (CTCs) are analyzed, the number of available cells is limited.

As described above, the present technology can increase a ratio of emulsion particles each containing one fine particle (e.g., cell). Therefore, the present technology is extremely effective in performing single cell analysis using an emulsion.

Further, in single cell analysis using an emulsion in which each emulsion particle contains one cell, a target cell group for single cell analysis is generally sorted by using a cell sorting device such as, for example, a cell sorter to purify target cells before the emulsion is formed. Therefore, in order to perform the single cell analysis, a cell sorter is required in addition to an emulsification device. Increase in the number of devices to be used is not desirable in view of cost, for example. Further, in order to form the emulsion, a cell sorting step is required in addition to an emulsion forming step. Increase in the number of steps is not desirable in view of time and cost, for example.

There is a technology of performing single cell sorting into a well plate on the basis of a detection signal by using a cell sorter. However, the number of wells in the well plate is up to 384, and therefore an analysis scale and throughput are low. Further, a sort nozzle cannot sort cells while moving between wells, and thus target cells flowing during this period are lost.

In the method of the present technology, the fine particles determined as the particles to be collected in the determination step can be collected in an emulsion state. Therefore, the present technology can form an emulsion without separately performing the cell sorting step. Further, the method of the present technology can increase a collection rate of the particles to be collected.

In a preferred embodiment of the present technology, an emulsion is formed by carrying out the method of collecting fine particles, and at least a part of droplets forming the emulsion (hereinafter, also referred to as "emulsion particles") contains one of the particles to be collected. More preferably, among all the droplets forming the emulsion, preferably 70% or more, more preferably 80% or more, and still more preferably 90% or more of the droplets contain one particle to be collected. The method of collecting fine particles according to the present technology can form an emulsion containing emulsion particles each containing one particle to be collected at a high ratio as described above.

Therefore, the present technology also provides a method of producing an emulsion in which each emulsion particle contains one fine particle. Steps included in the production method may be the same as the steps of the method of collecting fine particles.

Further, the present technology also provides an emulsion in which a ratio of the number of emulsion particles each containing one cell to the total number of emulsion particles is 70% or more. The ratio may be preferably 75% or more, and more preferably 80% or more, 85% or more, or 90% or more. As described above, the present technology provides an emulsion containing one emulsion particle at an extremely high content ratio.

In the channel structure of the fine particle sorting mechanism used in the method of the present technology, the main channel and the collection channel are connected via the connection channel, and the connection channel is connected to the liquid supply channel. By supplying the second liquid from the liquid supply channel to the connection channel, it is possible to prevent the first liquid flowing through the main channel from entering the collection channel, and it is also possible to introduce the first liquid into the collection channel as necessary. For example, the first liquid is introduced into the collection channel only in a case where the particle to be collected reaches near the connection channel. Therefore, the particle to be collected can be introduced into the collection channel while being contained in the first liquid.

By carrying out the method of collecting fine particles according to the present technology in accordance with a preferred embodiment of the present technology, an emulsion containing the second liquid as a dispersion medium and the first liquid as a dispersoid is formed in the collection channel. The emulsion formed by the method of collecting fine particles according to the present technology contains emulsion particles each containing one particle to be collected at a high content ratio with respect to the total emulsion particles. Therefore, the present technology is applicable even in a case where the number of cells to be analyzed is small.

A kinematic viscosity of the second liquid is preferably 1/1000 times to 1000 times, more preferably 1/100 times to 100 times, still more preferably 1/10 times to 10 times, further more preferably 1/5 times to 5 times, and particularly preferably 1/2 times to 2 times as much as the kinematic viscosity of the first liquid. In the present technology, the first liquid and the second liquid preferably have substantially the same kinematic viscosity. This makes it easy to form an emulsion.

Both densities of the first liquid and the second liquid at 25°C can be, for example, 0.5 g/cm³ to 5 g/cm³, preferably 0.6 g/cm³ to 4 g/cm³, and more preferably 0.7 g/cm³ to 3 g/cm³.

Further, the density of the second liquid is preferably 1/100 times to 100 times, more preferably 1/10 times to 10 times, still more preferably 1/5 times to 5 times, and particularly preferably 1/2 times to 2 times as much as that of the first liquid. In the present technology, the first liquid and the second liquid preferably have substantially the same density. This makes it easy to form an emulsion.

The kinematic viscosities of the first liquid and the second liquid at 25°C can be, for example, 0.3 cSt to 5 cSt, preferably 0.4 cSt to 4 cSt, and more preferably 0.5 cSt to 3 cSt.

Because the first liquid and the second liquid have the above physical properties, an emulsion is easily formed in the collection channel. Further, such physical properties make it easier for those liquids to flow through a microchannel.

In one embodiment of the present technology, the first liquid may be a hydrophilic liquid and the second liquid may be a hydrophobic liquid. In this embodiment, an emulsion containing the hydrophobic liquid as the dispersion medium and the hydrophilic liquid as the dispersoid can be formed in the collection channel. For example, it is desirable that biological particles such as cells exist in the hydrophilic liquid such as a buffer or culture solution. Therefore, this embodiment is suitable to collect fine particles, particularly biological particles, and more particularly cells, which desirably exist in the hydrophilic liquid.

The hydrophilic liquid contains, for example, water and a liquid that is miscible with water. For example, the hydrophilic liquid may be a liquid containing, as a main component, one or a mixture of two or more selected from the group consisting of water, hydrophilic alcohols, hydrophilic ethers, ketones, nitrile solvents, dimethyl sulfoxide, and N,N-dimethylformamide. In the present specification, the main component refers to a component that accounts for, for example, 50% by mass or more, particularly 60% by mass or more, more particularly 70% by mass or more, still more particularly 80% by mass or more, 85% by mass or more, or 90% by mass or more of the liquid. Examples of the hydrophilic alcohols include ethanol, methanol, propanol, and glycerin. Examples of the hydrophilic ethers include tetrahydrofuran, polyethylene oxide, and 1,4-dioxane. Examples of the ketones include acetone and methyl ethyl ketone. Examples of the nitrile solvents include acetonitrile.

The hydrophilic liquid may preferably be a liquid containing water as a main component and may be, for example, water, an aqueous solution, or aqueous dispersion. The hydrophilic liquid may be, for example, a sheath fluid and/or sample fluid. The hydrophilic liquid is preferably a hydrophilic liquid that does not adversely affect fine particles (e.g., biological particles, in particular, cells).

The hydrophilic liquid may be, for example, a liquid containing biomolecules. The biomolecules may be, for example, one or a combination of two or more selected from amino acids, peptides, and proteins.

Further, the hydrophilic liquid may contain, for example, a surfactant, in particular, a nonionic surfactant. Examples of the nonionic surfactant include, for example, a triblock copolymer of polyethylene oxide and polypropylene oxide, and the triblock copolymer is also referred to as "poloxamer" or "Pluronic surfactant". A more specific example of the Pluronic surfactant is Pluronic (trademark) F68.

Examples of the hydrophilic liquid include a culture solution and a buffer, but are not limited thereto. The buffer is preferably a Good buffer.

By using the culture solution as the hydrophilic liquid, cells collected as the particles to be collected can be cultured while being held in the emulsion particles.

Further, in a case where the hydrophilic liquid (in particular, the sheath fluid) contains a cell stimulating component, the cells collected as the particles to be collected can be stimulated while being held in the emulsion particles. Furthermore, characteristics (e.g., morphology and the like) of the stimulated cells can be observed by using a microscope or the like.

Moreover, the hydrophilic liquid (e.g., the sheath fluid or the sample fluid) may have an assay system that allows a response to cell stimulation to be observed. The assay system can, for example, optically detect the response from the cells collected as the particles to be collected while the cells are being held in the emulsion particles. The assay system is preferably a wash-free assay system and is preferably, for example, a system that uses fluorescence resonance energy transfer (FRET), bioluminescence resonance energy transfer (BRET), or the like.

As described above, in a case where the fine particles are biological particles (in particular, cells) in the present technology, it is possible to perform various kinds of analysis of single biological particles (in particular, single cell analysis such as, for example, single cell imaging).

A density of the hydrophilic liquid at 25°C can be, for example, 0.5 g/cm³ to 5 g/cm³, preferably 0.6 g/cm³ to 4 g/cm³, and more preferably 0.7 g/cm³ to 3 g/cm³.

A kinematic viscosity of the hydrophilic liquid at 25°C can be, for example, 0.3 cSt to 5 cSt, preferably 0.4 cSt to 4 cSt, and more preferably 0.5 cSt to 3 cSt.

The hydrophilic liquid has the above physical properties and therefore easily flows through the microchannel. Further, an emulsion is easily formed in the collection channel.

The hydrophobic liquid may be any liquid selected from liquids that are immiscible with the hydrophilic liquid. The hydrophobic liquid may be a liquid containing, as a main component, one or a mixture of two or more selected from the group consisting of, for example, aliphatic hydrocarbons, fluorine-based oils, a monomer or polymer containing fluorine atoms, silicone oils, aromatic hydrocarbons, aliphatic monohydric alcohols (e.g., n-octanol or the like), and polysaccharides fluoride.

The aliphatic hydrocarbon is preferably an aliphatic hydrocarbon having 7 or more and 30 or less carbon atoms. Because the number of carbon atoms is 7 or more and 30 or less, a kinematic viscosity of the hydrophobic liquid is suitable to flow through the microchannel. Examples of aliphatic hydrocarbons include: mineral oils; oils derived from animals and plants such as squalane oil and olive oil; paraffinic hydrocarbons having 10 to 20 carbon atoms such as decane and hexadecane; and olefinic hydrocarbons having 10 to 20 carbon atoms.

In the present technology, the hydrophobic liquid is preferably a fluorine-based oil in view of favorable immiscibility with the hydrophilic liquid. Examples of the fluorine-based oil include perfluorocarbon (PFC), perfluoropolyether (PFPE), and hydrofluoroether (HFE). Examples of perfluorocarbon include Fluorinert (trademark) FC40 and Fluorinert FC-770 (manufactured by 3M). Examples of perfluoropolyether include Krytox (manufactured by DuPont). Examples of hydrofluoroether include HFE7500 (manufactured by 3M).

A density of the hydrophobic liquid at 25°C can be, for example, 0.5 g/cm³ to 5 g/cm³, preferably 0.6 g/cm³ to 4 g/cm³, and more preferably 0.7 g/cm³ to 3 g/cm³.

A kinematic viscosity of the hydrophobic liquid at 25°C can be, for example, 0.3 cSt to 5 cSt, preferably 0.4 cSt to 4 cSt, and more preferably 0.5 cSt to 3 cSt.

Because the hydrophobic liquid has the above physical properties, an emulsion is easily formed in the collection channel. For example, in a case where the density or kinematic viscosity is too high, a possibility that the liquid may not smoothly flow through the connection channel increases.

In a preferred embodiment of the present technology, one or both of the first liquid and the second liquid can contain a surfactant. In particular, one or both of the hydrophobic liquid and the hydrophilic liquid contain the surfactant, and more particularly the hydrophobic liquid contains the surfactant. The surfactant makes it easier to form emulsion particles and also makes it possible to stably maintain the emulsion particles. Examples of the surfactant include nonionic surfactants and fluorine-based surfactants. Examples of the nonionic surfactants include Span80 and Abil EM, but are not limited thereto. The kind of surfactant may be appropriately selected by a person skilled in the art. Examples of the fluorine-based surfactants include perfluoropolyether-based surfactants and pseudosurfactants. Examples of the former surfactants include Krytox (manufactured by DuPont), and examples of the latter surfactants include perfluorooctanol.

The surfactant can exist in, for example, the hydrophobic liquid at a concentration equal to or more than a critical micelle concentration of the surfactant. The critical micelle concentration can be, for example, 1 µM to 1000 µM, and, in particular, 10 µM to 100 mM. Further, an interfacial tension of the surfactant may be, for example, 40 mN/m or less, and, in particular, 20 mN/m or less.

In another embodiment of the present technology, the first liquid may be a hydrophobic liquid and the second liquid may be a hydrophilic liquid.

In this embodiment, an emulsion containing the hydrophilic liquid as the dispersion medium and the hydrophobic liquid as the dispersoid can be formed in the collection channel. The present technology may be used to collect fine particles into the emulsion. Examples of the hydrophobic liquid and the hydrophilic liquid are as described above.

Further, this embodiment is applicable to a case where, for example, only target fine particles are collected from an emulsion in which the hydrophobic liquid and the hydrophilic liquid serve as the dispersion medium and the dispersoid, respectively, and emulsion particles contain fine particles.

Further, the assay system that can be used in the present technology can be not only a system in which fine particles fluoresce but also a system in which emulsion particles fluoresce. Therefore, in order to collect emulsion particles containing fine particles, the determination may be performed on the fine particles, the emulsion particles, or both the fine particles and emulsion particles in the determination step. As described above, in the present technology, whether or not the fine particles or emulsion particles are the particles to be collected may be determined on the basis of information obtained from the fine particles and/or emulsion particles.

According to a preferred embodiment of the present technology, the flow step, the determination step, and the collection step are performed while the second liquid is being supplied from the liquid supply channel to the connection channel. Therefore, the connection channel is filled with the second liquid. This makes it possible to prevent the first liquid from unnecessarily entering the collection channel.

### (2) First example of first embodiment

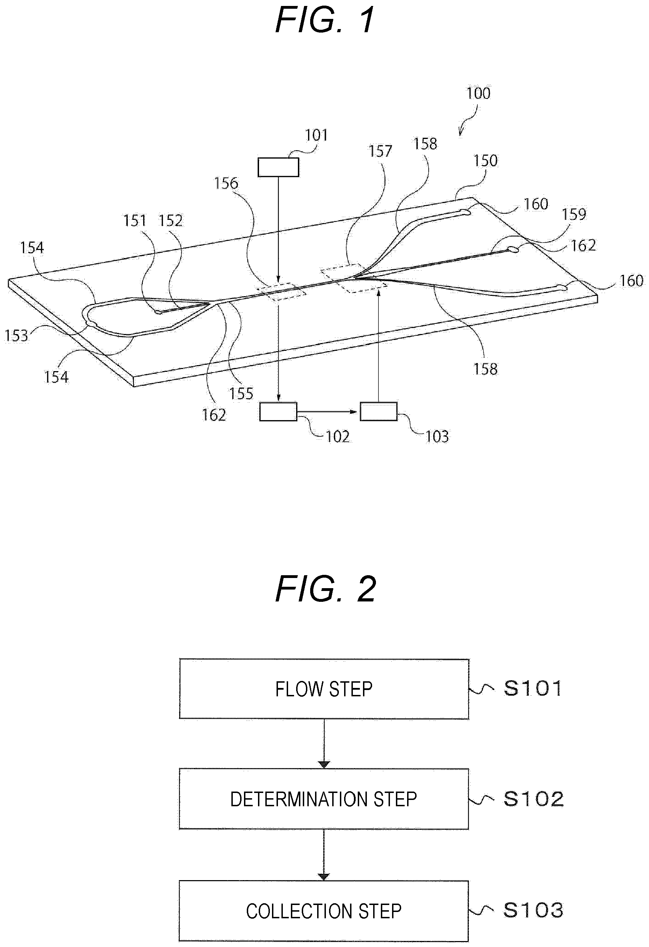

The method of collecting fine particles according to the present technology is performed by using a fine particle sorting mechanism. Hereinbelow, an example of one embodiment of the method of collecting fine particles according to the present technology will be described with reference to Fig. 1 illustrating a microchip for sorting fine particles that is a configuration example of the fine particle sorting mechanism used in the method according to the present technology and Fig. 2 showing an example of a flow of the method of collecting fine particles according to the present technology.

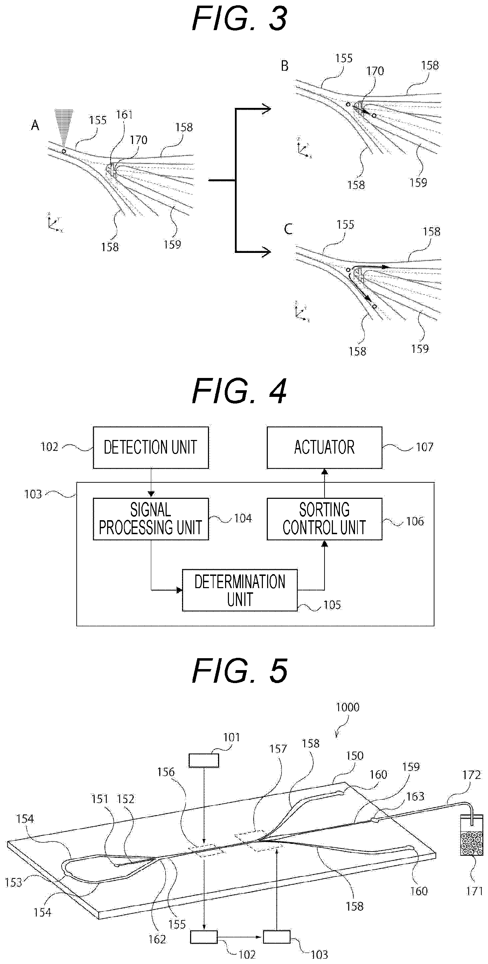

As illustrated in Fig. 1, a microchip 150 for sorting fine particles used in the method of the present technology has a main channel 155 through which fine particles flow and a collection channel 159 into which particles to be collected are collected from among the fine particles. The microchip 150 for sorting fine particles has a particle sorting portion 157. Fig. 3 illustrates an enlarged view of the particle sorting portion 157. As illustrated in A of Fig. 3, the particle sorting portion 157 has a connection channel 170 that connects the main channel 155 and the collection channel 159. The connection channel 170 is connected to liquid supply channels 161 capable of supplying a liquid to the connection channel 170. As described above, the microchip 150 for sorting fine particles has a channel structure including the main channel 155, the collection channel 159, the connection channel 170, and the liquid supply channels 161.

Further, as illustrated in Fig. 1, the microchip 150 for sorting fine particles forms a part of a device 100 for collecting fine particles including, in addition to the microchip, a light irradiation unit 101, a detection unit 102, and a control unit 103. As shown in Fig. 4, the control unit 103 can include a signal processing unit 104, a determination unit 105, and a sorting control unit 106.

As shown in Fig. 2, the method of the present technology includes, in the microchip 150 for sorting fine particles, a flow step S101 of causing a first liquid containing fine particles to flow through the main channel 155, a determination step S102 of determining whether or not the fine particles flowing through the main channel 155 are particles to be collected, and a collection step S103 of collecting the particles to be collected into the collection channel 159.

Each step will be described below.

### (2-1) Flow step

In the flow step S101, the first liquid containing the fine particles flows through the main channel 155. The first liquid flows through the main channel 155 from a joining portion 162 toward the particle sorting portion 157. The first liquid may be a laminar flow of a sample fluid containing the fine particles and a sheath fluid and may particularly be a laminar flow in which the sample fluid is surrounded by the sheath fluid. A channel structure for forming the laminar flow will be described below.

The microchip 150 for sorting fine particles has a sample fluid inlet 151 and a sheath fluid inlet 153. The sample fluid containing the fine particles and the sheath fluid containing no fine particle are introduced from those inlets into a sample fluid channel 152 and sheath fluid channels 154, respectively.

The microchip 150 for sorting fine particles has a channel structure in which the sample channel 142 through which the sample fluid flows and the sheath fluid channels 154 through which the sheath fluid flows join at the joining portion 162 to form the main channel 155. The sample fluid and the sheath fluid join at the joining portion 162 to form, for example, a laminar flow in which the sample fluid is surrounded by the sheath fluid. The fine particles are preferably arranged substantially in a row in the laminar flow. As described above, in the present technology, the channel structure forms a laminar flow containing the fine particles flowing substantially in a row.

The laminar flow flows through the main channel 155 toward the particle sorting portion 157. The fine particles preferably flow through the main channel 155 in a row. This makes it easy to, in light irradiation in a detection region 156 described below, distinguish between light generated by light irradiation for a single fine particle and light generated by light irradiation for other fine particles.

### (2-2) Determination step

In the determination step S102, it is determined whether or not each fine particle flowing through the main channel 155 is a particle to be collected. The determination can be made by the determination unit 105. The determination unit 105 can make the determination on the basis of light generated by light irradiation for the fine particle by the light irradiation unit 101. An example of the determination step S102 will be described in more detail below.

In the determination step S102, the light irradiation unit 101 irradiates each fine particle flowing through the main channel 155 (in particular, the detection region 156) in the microchip 150 for sorting fine particles with light (e.g., excitation light or the like), and the detection unit 102 detects light generated by the light irradiation. According to a characteristic of the light detected by the detection unit 102, the determination unit 105 included in the control unit 103 determines whether or not the fine particle is a particle to be collected. For example, the determination unit 105 can make the determination based on scattered light, fluorescence, or an image (e.g., a dark-field image, bright-field image, and/or the like). In the collection step S103 described below, the control unit 103 controls the flow in the microchip 150 for sorting fine particles, and therefore the particle to be collected is collected into the collection channel 159.

The light irradiation unit 101 irradiates the fine particle flowing through the channel in the microchip 150 for sorting fine particles with light (e.g., excitation light or the like). The light irradiation unit 101 can include a light source that emits light and an objective lens that condenses excitation light for the fine particle flowing in the detection region. The light source may be appropriately selected by a person skilled in the art in accordance with the purpose of analysis and may be, for example, a laser diode, SHG laser, solid-state laser, gas laser, high-intensity LED, or halogen lamp, or may be a combination of two or more thereof. The light irradiation unit may include not only the light source and the objective lens but also other optical elements as necessary.

### (Determination on sorting target based on fluorescence signal and/or scattered light signal)

In one embodiment of the present technology, the detection unit 102 detects scattered light and/or fluorescence generated from the fine particle by light irradiation by the light irradiation unit 101. The detection unit 102 can include a detector and a condenser lens that condenses fluorescence and/or scattered light generated from the fine particle. The detector can be a PMT, photodiode, CCD, CMOS, or the like, but is not limited thereto. The detection unit 102 may include not only the condenser lens and the detector but also other optical elements as necessary. The detection unit 102 can further include, for example, a diffraction unit. Examples of optical components included in the diffraction unit include a grating, prism, and optical filter. The diffraction unit can detect, for example, light having a wavelength to be detected separately from light having other wavelengths. The detection unit 102 can convert the detected light into an analog electric signal by photoelectric conversion. The detection unit 102 can further convert the analog electric signal into a digital electric signal by AD conversion.

The signal processing unit 104 included in the control unit 103 can process a waveform of the digital electric signal obtained by the detection unit 102 to generate information (data) regarding the characteristic of the light used for the determination by the determination unit 105. The signal processing unit 104 can acquire, as the information regarding the characteristic of the light, for example, one, two, or three of a width of the waveform, a height of the waveform, and an area of the waveform from the waveform of the digital electric signal. Further, the information regarding the characteristic of the light may include, for example, the time when the light has been detected. The above processing by the signal processing unit 104 can be performed particularly in an embodiment in which the scattered light and/or fluorescence is detected.

The determination unit 105 included in the control unit 103 determines whether or not the fine particle is the particle to be collected on the basis of the light generated by irradiating the fine particle flowing through the channel with light.

In the embodiment in which the scattered light and/or fluorescence is detected, the waveform of the digital electric signal obtained by the detection unit 102 is processed by the control unit 103, and, on the basis of the information regarding the characteristic of the light generated by the processing, the determination unit 105 determines whether or not the fine particle is the particle to be collected. For example, characteristics of an outer shape and/or internal structure of the fine particle may be specified in the determination based on the scattered light, and whether or not the fine particle is the particle to be collected may be determined on the basis of the characteristics. Further, in a case where the fine particle such as, for example, a cell is pretreated in advance, it is also possible to determine whether or not the fine particle is the particle to be collected on the basis of a characteristic similar to a characteristic used in flow cytometry. Further, in a case where, for example, the fine particle such as a cell is labeled with an antibody or dye (in particular, a fluorescent dye), it is also possible to determine whether or not the fine particle is the particle to be collected on the basis of a characteristic of a surface antigen of the fine particle.

### (Determination on sorting target based on bright-field image)

In another embodiment of the present technology, the detection unit 102 may acquire a bright-field image generated by light irradiation by the light irradiation unit 101. In this embodiment, the light irradiation unit 101 can include, for example, a halogen lamp, and the detection unit 102 can include a CCD or CMOS. For example, the halogen lamp can irradiate the fine particle with light, and the CCD or CMOS can acquire a bright-field image of the irradiated fine particle.

In the embodiment in which the bright-field image is acquired, the determination unit 105 included in the control unit 103 determines whether or not the fine particle is the particle to be collected on the basis of the acquired bright-field image. For example, it is possible to determine whether or not the fine particle is the particle to be collected on the basis of one or a combination of two or more of morphology, size, and color of the fine particle (in particular, a cell).

### (Determination on sorting target based on dark-field image)

In still another embodiment of the present technology, the detection unit 102 may acquire a dark-field image generated by light irradiation by the light irradiation unit 101. In this embodiment, the light irradiation unit 101 can include, for example, a laser light source, and the detection unit 102 can include a CCD or CMOS. For example, the laser can irradiate the fine particle with light, and the CCD or CMOS can acquire a dark-field image (e.g., a fluorescent image) of the irradiated fine particle.

In the embodiment in which the dark-field image is acquired, the determination unit 105 included in the control unit 103 determines whether or not the fine particle is the particle to be collected on the basis of the acquired dark-field image. For example, it is possible to determine whether or not the fine particle is the particle to be collected on the basis of one or a combination of two or more of morphology, size, and color of the fine particle (in particular, a cell).

In any of the above-described "Determination on sorting target based on fluorescence signal and/or scattered light signal", "Determination on sorting target based on bright-field image", and "Determination on sorting target based on dark-field image", the detection unit 102 may be, for example, an imaging element in which a substrate in which a CMOS sensor is incorporated and a substrate in which a digital signal processor (DSP) is incorporated are laminated. In a case where the DSP of the imaging element is operated as a machine learning unit, the imaging element can operate as a so-called AI sensor. The detection unit 102 including the imaging element can determine whether or not the fine particle is the particle to be collected on the basis of, for example, a learning model. Further, the learning model may be updated in real time while the method according to the present technology is being performed. For example, the DSP can perform machine learning processing during reset of a pixel array unit in the CMOS sensor, during exposure of the pixel array unit, or during reading of a pixel signal from each unit pixel of the pixel array unit. An example of the imaging element that operates as an AI sensor is, for example, an imaging device disclosed in WO 2018/051809. In a case where the AI sensor is used as the imaging element, raw data acquired from an image array is learned as it is, and therefore sorting determination processing is performed at a high speed.

The determination can be made depending on, for example, whether or not the information regarding the characteristic of the light meets a criterion specified in advance. The criterion can be a criterion indicating that the fine particle is the particle to be collected. The criterion may be appropriately set by a person skilled in the art and can be a criterion regarding a characteristic of light, such as a criterion used in a technical field such as, for example, flow cytometry.

One position in the detection region 156 may be irradiated with a ray of light, or a plurality of positions in the detection region 156 may be irradiated with rays of light, respectively. For example, the microchip 150 can be configured to irradiate each of two different positions in the detection region 156 with light (i.e., there are two positions irradiated with light in the detection region 156). In this case, for example, it is possible to determine whether or not the fine particle is the particle to be collected on the basis of light (e.g., fluorescence, scattered light, and/or the like) generated by irradiating the fine particle at one position with light. Further, it is also possible to calculate a velocity of the fine particle in the channel on the basis of a difference between a detection time of the light generated by the light irradiation at the one position and a detection time of light generated by the light irradiation at the other position. For the calculation, a distance between the two irradiation positions may be determined in advance, and the velocity of the fine particle can be determined on the basis of the difference between the two detection times and the distance. Further, on the basis of the velocity, it is possible to accurately predict a time of arrival at the particle sorting portion 157 described below. Because the time of arrival is accurately predicted, it is possible to optimize a timing of forming a flow that enters the collection channel 159. Further, in a case where a difference between the time of arrival of a certain fine particle at the particle sorting portion 157 and a time of arrival of a fine particle before or after the certain fine particle at the particle sorting portion 157 is equal to or less than a predetermined threshold, it is also possible to determine that the certain fine particle is not to be collected.

In a case where the certain fine particle and the fine particle before or after the certain fine particle have a small distance, a possibility that the fine particle before or after the certain fine particle is collected together with the certain fine particle at the time of suction of the certain fine particle increases. In a case where there is a high possibility that the fine particles are collected together, it is determined that the certain fine particle is not to be collected. This makes it possible to prevent the fine particle before or after the certain fine particle from being collected. Therefore, it is possible to increase purity of the target fine particles among the collected fine particles. Specific examples of the microchip in which two different positions in the detection region 156 are irradiated with rays of light, respectively, and a device including the microchip are disclosed in, for example, Japanese Patent Application Laid-Open No. 2014-202573.

Note that the control unit 103 may control the light irradiation by the light irradiation unit 101 and/or the light detection by the detection unit 102. Further, the control unit 103 can control drive of a pump for supplying a fluid into the microchip 150 for sorting fine particles. The control unit 103 may include, for example, a hard disk in which a program for causing the device for collecting fine particles to execute the method of collecting fine particles according to the present technology and an OS are stored, a CPU, and a memory. For example, functions of the control unit 103 can be realized by a general-purpose computer. The program may be recorded on a recording medium such as, for example, a microSD memory card, SD memory card, or flash memory. A drive (not illustrated) included in the device 100 for collecting fine particles may read the program recorded on the recording medium, and the control unit 103 may cause the device 100 for collecting fine particles to execute the method of collecting fine particles according to the present technology in accordance with the read program.

### (2-3) Collection step

In the collection step S103, the fine particle determined as the particle to be collected in the determination step S102 is collected into the collection channel 159. In the collection step S103, the particle to be collected is collected into the second liquid that is immiscible with the first liquid in the collection channel while being contained in the first liquid. Therefore, an emulsion containing the second liquid as the dispersion medium and the first liquid as the dispersoid can be formed in the collection channel 159, and each emulsion particle of the emulsion contains one particle to be collected. Therefore, the emulsion is suitable for single cell analysis.

The collection step will be described in more detail below.

The collection step S103 is performed in the particle sorting portion 157 in the microchip 150. In the particle sorting portion 157, the laminar flow that has flowed through the main channel 155 separately flows into two waste channels 158. The particle sorting portion 157 illustrated in Fig. 1 has the two waste channels 158, but the number of bifurcating channels is not limited to two. The particle sorting portion 157 can be provided with, for example, one or a plurality of (e.g., two, three, four, or the like) bifurcating channels. The bifurcating channels may be configured to bifurcate in a Y shape on a plane as illustrated in Fig. 1 or may be configured to three-dimensionally bifurcate.

In the particle sorting portion 157, a flow entering the collection channel 159 from the main channel 155 through the connection channel 170 is formed only in a case where the particle to be collected flows, and the particle to be collected is collected into the collection channel 159. Fig. 3 illustrates an enlarged view of the particle sorting portion 157. As illustrated in Fig. 3A, the main channel 155 and the collection channel 159 communicate with each other via the connection channel 170 coaxial with the main channel 155. As illustrated in Fig. 3B, the particle to be collected flows into the collection channel 159 through the connection channel 170. As illustrated in Fig. 3C, a fine particle that is not the particle to be collected flows into one of the waste channels 158.

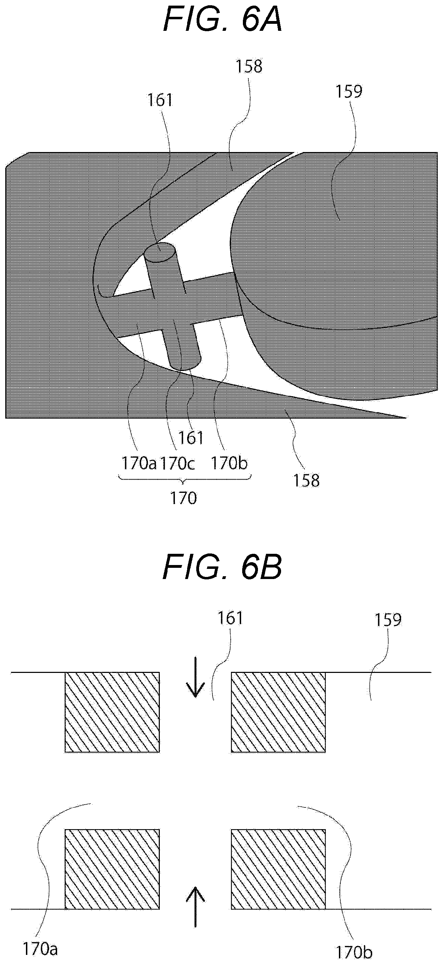

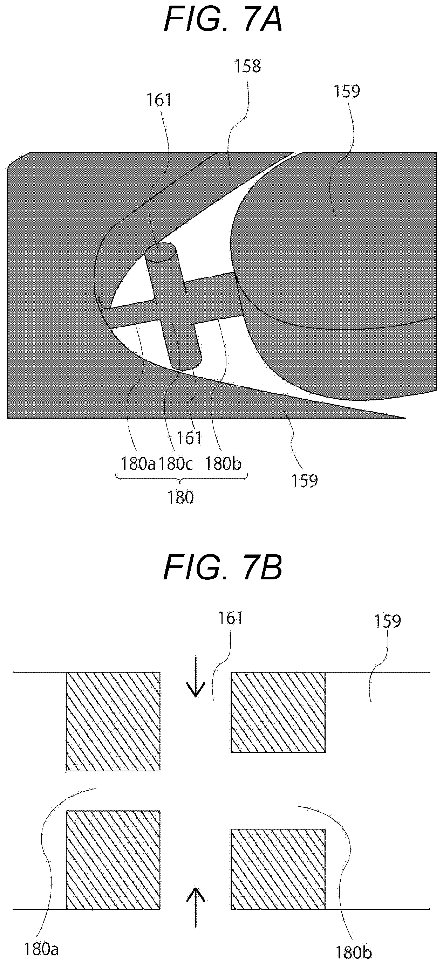

Figs. 6A and 6B illustrate enlarged views of the connection channel 170 and the vicinity thereof. Fig. 6A is a schematic perspective view of the connection channel 170 and the vicinity thereof. Fig. 7B is a schematic cross-sectional view in a plane passing through a center line of the liquid supply channels 161 and a center line of the connection channel 170. The connection channel 170 has a channel 170a on the detection region 156 side (hereinafter, also referred to as "upstream connection channel 170a"), a channel 170b on the collection channel 159 side (hereinafter, also referred to as "downstream connection channel 170b"), and a connection portion 170c between the connection channel 170 and the liquid supply channels 161. The liquid supply channels 161 are provided to be substantially perpendicular to an axis of the channel of the connection channel 170. In Figs. 6A and 6B, the two liquid supply channels 161 are provided to face each other at substantially a center position of the connection channel 170. However, only one liquid supply channel may be provided.

A shape and dimensions of a cross section of the upstream connection channel 170a may be the same as the shape and dimensions of the downstream connection channel 170b. For example, as illustrated in Figs. 6A and 6B, both the cross section of the upstream connection channel 120a and the cross section of the downstream connection channel 120b may have substantially a circular shape having the same dimensions. Alternatively, both of those two cross sections may have a rectangular shape (e.g., square, rectangle, or the like) having the same dimensions.

The second liquid is supplied from the two liquid supply channels 161 to the connection channel 170 as indicated by arrows in Fig. 6B. The second liquid flows from the connection portion 170c to both the upstream connection channel 170a and the downstream connection channel 170b.

In a case where the collection step is not performed, the second liquid flows as follows.

The second liquid that has flowed into the upstream connection channel 170a flows out from a connection surface between the connection channel 170 and the main channel 155 and then separately flows into the two waste channels 158. Because the second liquid flows out from the connection surface as described above, it is possible to prevent the first liquid and a fine particle that do not need to be collected into the collection channel 159 from entering the collection channel 159 through the connection channel 170.

The second liquid that has flowed into the downstream connection channel 170b flows into the collection channel 159. Therefore, the inside of the collection channel 159 is filled with the second liquid, and the second liquid serves as, for example, a dispersion medium for forming an emulsion.

Also in a case where the collection step is performed, the second liquid can be supplied from the two liquid supply channels 161 to the connection channel 170. However, a flow flowing from the main channel 155 into the collection channel 159 through the connection channel 170 is formed due to a pressure fluctuation in the collection channel 159, in particular, by generating a negative pressure in the collection channel 159. That is, a flow flowing from the main channel 155 into the collection channel 159 through the upstream connection channel 170a, the connection portion 170c, and the downstream connection channel 170b in this order is formed. Therefore, the particle to be collected is collected into the second liquid in the collection channel 159 while being surrounded by the first liquid. By performing the collection step, it is possible to form, for example, an emulsion in the collection channel 159 or in, for example, a container connected to an end 163 of the collection channel via a channel.

The shape and/or dimensions of the cross section of the upstream connection channel 120a may be different from the shape and/or dimensions of the downstream connection channel 120b. An example of those two channels having different dimensions is illustrated in Figs. 7A and 7B. As illustrated in Figs. 7A and 7B, a connection channel 180 includes a channel 180a on the detection region 156 side (hereinafter, also referred to as "upstream connection channel 180a"), a channel 180b on the collection channel 159 side (hereinafter, also referred to as "downstream connection channel 180b"), and a connection portion 180c between the connection channel 180 and the liquid supply channels 161. Both a cross section of the upstream connection channel 180a and a cross section of the downstream connection channel 180b have substantially a circular shape, but a diameter of the latter cross section is larger than a diameter of the former cross section. In a case where the diameter of the latter cross section is made larger than that of the former cross section, it is possible to more effectively prevent the particle to be collected that has already sorted in the collection channel 159 from being released to the main channel 155 through the connection channel 180 immediately after the above-described fine particle sorting operation using a negative pressure, as compared with a case where both the cross sections have the same diameter.

For example, in a case where both the cross section of the upstream connection channel 180a and the cross section of the downstream connection channel 180b have a rectangular shape, an area of the latter cross section is made larger than an area of the former cross section. Therefore, as described above, it is possible to more effectively prevent the already collected fine particle from being released to the main channel 155 through the connection channel 180.

In the collection step S103, the particle to be collected is collected into the collection channel through the connection channel due to a pressure fluctuation in the collection channel 159. The collection may be performed by, for example, generating a negative pressure in the collection channel 159 as described above. The negative pressure can be generated when, for example, an actuator 107 (in particular, a piezoelectric actuator) attached to the outside of the microchip 150 deforms a wall defining the collection channel 159. The negative pressure can form the flow entering the collection channel 159. The actuator 107 can be attached to the outside of the microchip 150 so as to generate the negative pressure, for example, so as to deform the wall of the collection channel 159. The deformation of the wall can change an inner space of the collection channel 159 and generate the negative pressure. The actuator 107 can be, for example, a piezoelectric actuator. When the particle to be collected is sucked into the collection channel 159, the sample fluid forming the laminar flow or the sample fluid and the sheath fluid forming the laminar flow can also flow into the collection channel 159. In this way, the particle to be collected is sorted in the particle sorting portion 157 and is collected into the collection channel 159.

The particle to be collected is collected into the second liquid that is immiscible with the first liquid in the collection channel 159 while being surrounded by the first liquid. Therefore, as described above, an emulsion containing the second liquid as the dispersion medium and the first liquid as the dispersoid is formed in the collection channel 159.

The connection channel 170 is provided with the liquid supply channels 161 in order to prevent a fine particle that is not the particle to be collected from entering the collection channel 159 through the connection channel 170. The second liquid that is immiscible with the liquid (sample fluid and sheath fluid) flowing through the main channel 155 is introduced into the connection channel 170 from the liquid supply channels 161.

Part of the second liquid introduced into the connection channel 170 forms a flow toward the main channel 155 from the connection channel 170. This prevents the fine particle other than the particle to be collected from entering the collection channel 159. Because of a flow of the first liquid flowing through the main channel 155 to the waste channels 158, the second liquid forming the flow toward the main channel 155 from the connection channel 170 flows through the waste channels 158 in a similar manner to the first liquid, without flowing through the main channel 155.

Note that the rest of the second liquid introduced into the connection channel 170 flows into the collection channel 159. Therefore, the inside of the collection channel 159 can be filled with the second liquid.

The collection channel 159 may be filled with the second liquid that is immiscible with the first liquid. In order to fill the inside of the collection channel 159 with the second liquid, the second liquid can be supplied from the liquid supply channels 161 to the connection channel 170. By the supply, the second liquid can flow from the connection channel 170 into the collection channel 159, and therefore the inside of the collection channel 159 can be filled with the second liquid.

The laminar flow that has flowed into the waste channels 158 can be discharged to the outside of the microchip at ends 160 of the waste channels. Further, the particle to be collected collected into the collection channel 159 can be discharged to the outside of the microchip at the end 161 of the collection channel.

As illustrated in, for example, Fig. 5, a container 171 can be connected to the end 163 of the collection channel via a channel such as a tube 172. As illustrated in Fig. 5, an emulsion containing the first liquid containing the particle to be collected as the dispersoid and the second liquid as the dispersion medium is collected into the container 171. As described above, according to one embodiment of the present technology, the device 100 for collecting fine particles may have a channel for collecting the emulsion containing the particle to be collected into the container.

Further, when the end 163 of the collection channel is closed and a collection operation is performed, a plurality of emulsion particles can be held in the collection channel 159. After the collection operation is completed, an assay such as, for example, single cell analysis can be continuously performed in the collection channel 159.

As described above, in the present technology, the main channel may bifurcate into the connection channel and the at least one waste channel. The at least one waste channel is a channel through which a fine particle other than the particle to be collected flows.

Further, as illustrated in Figs. 1 and 2, the main channel, the connection channel, and the collection channel may be linearly arranged in the microchip for sorting fine particles used in the method of the present technology. In a case where those three channels are arranged linearly (in particular, on the same axis), it is possible to more efficiently perform the collection step than in a case where, for example, the connection channel and the collection channel are arranged at an angle with respect to the main channel. For example, it is possible to reduce an amount of suction required to introduce the particle to be collected into the connection channel.

Further, as illustrated in Figs. 1 and 2, in the microchip for sorting fine particles used in the method of the present technology, the fine particles are arranged substantially in a row in the main channel and flow toward the connection channel. Therefore, it is also possible to reduce the amount of suction in the collection step.

Further, in the method of the present technology, the liquid supply channels supply a liquid (in particular, the second liquid) to the connection channel. Therefore, a flow flowing toward the main channel from a connection position between the liquid supply channels and the connection channel is formed in the connection channel. This makes it possible to prevent the liquid flowing through the main channel from entering the connection channel and also to prevent a fine particle other than the particle to be collected from flowing into the collection channel through the connection channel. When the collection step is performed, as described above, the first liquid containing one particle to be collected flows through the connection channel and is collected into the second liquid in the collection channel due to, for example, a negative pressure generated in the collection channel. Therefore, an emulsion particle containing one particle to be collected is formed in the second liquid.

Further, in the present technology, for example, the piezoelectric actuator is driven at an appropriate timing (e.g., when the fine particle arrives at the particle sorting portion 157), and therefore the fine particle determined as the particle to be collected in the determination step and a hydrophilic solution containing the particle to be collected are collected into the collection channel 159 to form an emulsion particle. In the determination step, for example, in a case where whether or not the fine particle is the particle to be collected is determined by using a peak signal and an area signal, it is also possible to determine whether the particle is a single fine particle (singlet), two bound fine particles (doublet), or three bound fine particles (triplet). This makes it possible to avoid formation of emulsion particles each containing two or more fine particles. Therefore, emulsion particles each containing one fine particle can be formed with high probability and high efficiency. Further, because it is possible to avoid formation of emulsion particles each containing two or more bound fine particles as described above, it is possible to omit an operation for removing a bound substance of two or more fine particles by using, for example, a cell sorter or the like before the emulsion formation operation.

On the contrary, it is possible to determine a characteristic of each of two or more fine particles that are close enough to be drawn into the connection channel at the same time in one collection operation. For example, it is possible to enclose two fine particles having the same characteristic in one emulsion particle or also enclose a combination of fine particles having different specified characteristics in one emulsion particle.

### (Example)

A microchip for sorting fine particles having the same channel structure as the microchip 150 for sorting fine particles illustrated in Fig. 1 was used to form an emulsion particle containing one fine particle as described below. Hereinafter, an operation for forming the emulsion particle will be described with reference to Fig. 1.

A piezoelectric element (piezoelectric actuator) was attached to an outer surface (in particular, an outer surface corresponding to an expanded part, which is near the connection portion to the connection channel 170 in the collection channel 159) of the microchip 150 for sorting fine particles so as to change a volume of the collection channel 159.

A hydrophilic sample fluid containing beads having a diameter of 10 µm was introduced into the sample fluid channel 152 from the sample fluid inlet 151, and a hydrophilic sheath fluid was introduced into the sheath fluid channels 154 from the sheath fluid inlet 153. The introduced hydrophilic sample fluid and the hydrophilic sheath fluid joined at the joining portion 162 to form a laminar flow in which the hydrophilic sample fluid was surrounded by the hydrophilic sheath fluid. The laminar flow contained the beads arranged substantially in a row and flowed through the main channel 155 toward the connection channel 170.

Further, a hydrophobic liquid was supplied from the liquid supply channels 161 to the connection channel 170 in parallel with the introduction of the hydrophilic sample fluid and the hydrophilic sheath fluid. The supply of the hydrophobic liquid from the liquid supply channels 161 to the connection channel 170 prevented the laminar flow from entering the collection channel 159 through the connection channel 170, and the collection channel 159 was filled with the hydrophobic liquid.

When each bead passed through an irradiation position of laser light with which the detection region 156 of the main channel 155 was irradiated, the bead was irradiated with laser light, thereby generating light. The generated light was detected, and whether to collect each bead was determined on the basis of a characteristic of the detected light.

The piezoelectric element was driven at a timing at which the bead determined to be collected arrived at the vicinity of the connection channel 170 to deform the lumen of the collection channel 159. Therefore, the bead was collected into the collection channel 159 through the connection channel 170. Operations for the collection were as follows: (i) deforming the collection channel 159 for 10 µsec to apply a negative pressure to the inside of the collection channel 159; (ii) holding the deformed state for 10 µsec; and (iii) releasing the negative pressure for 10 µsec to restore the deformation. By repeating the above operations (i) to (iii), an emulsion containing emulsion particles each containing one bead in the hydrophobic liquid was formed in the collection channel 159. The emulsion contained the hydrophobic liquid as the dispersion medium and the emulsion particles containing the hydrophilic liquid as the dispersoid.

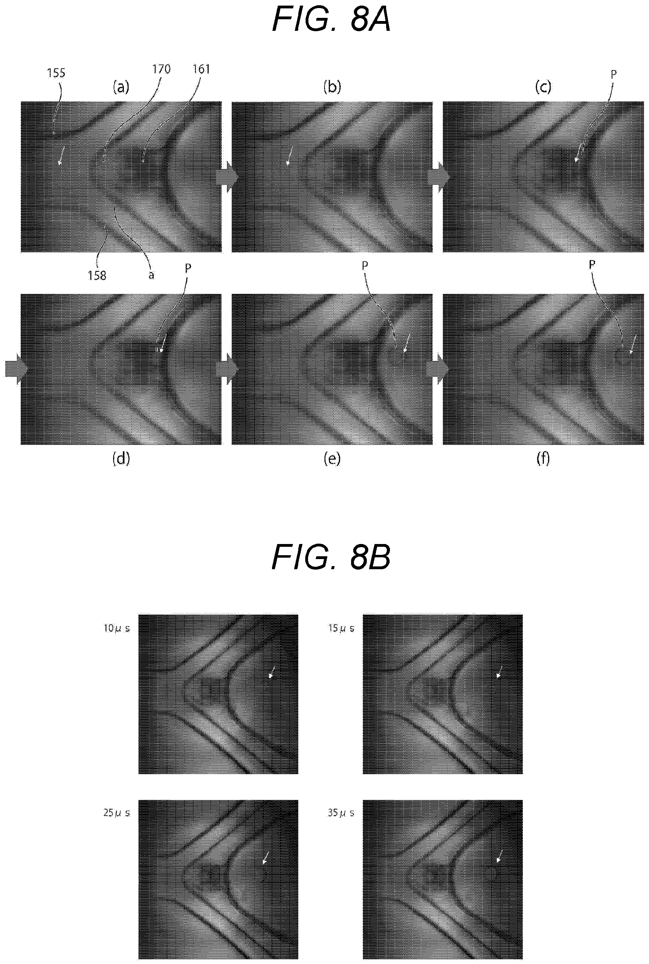

Fig. 8A shows that an emulsion particle containing one bead was formed in the collection channel 159 by performing the above operations (i) to (iii). Fig. 8A will be described below.
(a) of Fig. 8A is a photograph showing a state in which a bead (indicated by a white arrow) flowed toward the connection channel 170. At the point of Fig. 8A, the hydrophilic liquid flowed through the main channel 155 toward the particle sorting portion 157 and then flowed into the two waste channels 158 bifurcating from the main channel 155, without flowing into the connection channel 170. The hydrophobic liquid was supplied from the liquid supply channels 161 to the connection channel 170 and flowed into both the main channel 155 and the collection channel 159. The hydrophobic liquid that had flowed into the main channel 155 separately flowed into the two waste channels 158 immediately after flowing out from the connection channel 170 due to the flow of the hydrophilic liquid (flowed in the vicinity of a part indicated by a of Fig. 8A along walls of the waste channels 158).
(b) of Fig. 8A is a photograph showing a point at which the bead became closer to the connection channel. At that point, an operation for collecting the bead was started. That is, the piezoelectric element was driven to start deformation of the lumen of the collection channel 159.
(c) of Fig. 8A is a photograph showing a state in which the hydrophilic liquid flowed into the collection channel 159. It can be seen from the photograph that, at the point of Fig. 8C, the bead was surrounded by the hydrophilic liquid and the hydrophilic liquid was further surrounded by the hydrophobic liquid. That is, an emulsion particle P containing one bead was formed.
(d) of Fig. 8A is a photograph showing a state immediately before the emulsion particle P entered the collection channel 159 from the connection channel 170.
(e) of Fig. 8A is a photograph showing a state immediately after the emulsion particle P entered the collection channel 159. It can be confirmed that one bead was contained in the emulsion particle P.
(f) of Fig. 8A is a photograph showing that the emulsion particle P flowed further downstream in the ocean current channel 159.

As described above, an emulsion particle containing one bead was formed by the method according to the present technology.

It is possible to adjust a size of the emulsion particle by controlling a time period of each of the above operations (i) to (iii) and an amount of deformation. That is, the size of the emulsion particle in the collection channel can be controlled by a channel volume expanded by deforming the collection channel. Therefore, the size of the emulsion particle can be easily controlled by an amount of deformation of the piezoelectric element, that is, an electrical drive waveform of the piezoelectric element.

For example, in a case where the amount of deformation of the lumen of the collection channel is increased by the piezoelectric element, an amount of hydrophilic liquid to be sucked into the collection channel 159 increases, which makes it possible to increase the size of the emulsion particle. Meanwhile, in a case where the amount of deformation is reduced, the amount of hydrophilic liquid to be sucked into the collection channel 159 decreases, which makes it possible to reduce the size of the emulsion particle.

Further, the size of the emulsion particle can be further increased or decreased by further increasing or decreasing the holding time of the above operation (ii). This is shown in Fig. 8B. Fig. 8B is photographs showing the size of the emulsion particle in a case where the holding time of the above operation (ii) is 10 µsec, 15 µsec, 25 µsec, and 35 µsec. It can be seen from those photographs that the size of the emulsion particle can be adjusted by changing the holding time in the above operation (ii).

Further, it is also possible to adjust the drawing of the emulsion particle by adjusting a volume of the connection channel 170 (in particular, each volume of the upstream connection channel 170a, the connection portion 170c, and the downstream connection channel 170b).

In the example described above, the first liquid flowing through the main channel is prevented from entering the connection channel by supplying the second liquid from the liquid supply channels to the connection channel. In the present technology, in order to prevent the first liquid flowing through the main channel from entering the connection channel, a valve for preventing the first liquid from entering the collection channel may be provided in the connection channel. Only when the collection step is performed, the valve can be released and the first liquid can flow into the collection channel through the connection channel.

### (2-4) Other steps

The method of collecting fine particles according to the present technology may further include other steps in addition to the flow step, the determination step, and the collection step described above. Examples of the other steps will be described below.

### (2-4-1) Culture step

According to one preferred embodiment of the present technology, the fine particles are cells or cell aggregation, and the first liquid is a culture solution of the fine particles. In this embodiment, it is possible to form an emulsion containing the culture solution as the dispersoid and the second liquid that is immiscible with the culture solution as the dispersion medium. Each emulsion particle formed by the dispersoid may contain one cell or cell aggregation. Therefore, it is possible to culture one cell or cell aggregation in each emulsion particle. As described above, in this embodiment, the method of collecting fine particles can further include a culture step of culturing the particle to be collected (that is, cell or cell aggregation) collected in the collection step in the emulsion particle formed from the culture solution. The kind of the culture solution can be appropriately selected by a person skilled in the art in accordance with a cell to be cultured. This embodiment is suitable in a case where, for example, each cell or cell aggregation is required to be separately cultured in single cell analysis.

### (2-4-2) Destruction step

According to one preferred embodiment of the present technology, the fine particles can be cells, cell aggregation, or synthetic particles, and the fine particles can be destroyed after the collection step. In this embodiment, an emulsion can be formed in the collection step, and the cell, cell aggregation, or synthetic particle in each emulsion particle forming the emulsion can be destroyed. As described above, the method of collecting fine particles according to the present technology can include a destruction step of destroying the collected fine particle after the collection step.

According to other preferred embodiments of the present technology, a particle other than the cell, cell aggregation, or synthetic particle may be destroyed. That is, the method of collecting fine particles according to the present technology can include a destruction step of destroying the collected particle to be collected after the collection step.

The destruction can preferably be performed while the emulsion particle is being held. Therefore, for example, a cell component (e.g., an intracellular component, cell membrane component, cell wall component, or the like) or a component contained in a synthetic particle can be released into the emulsion particle, and the cell component or the component can be processed separately from components of other fine particles. The processing can be, for example, analysis, separation, or amplification of the cell component or the component. Examples of the cell component or the component include DNA, RNA, proteins, peptides, amino acids, lipids, saccharides, and organelles, but are not limited thereto. For example, in a case where the cell component or the component is DNA, it is possible to prepare a sample for DNA barcoding by performing the disruption step or by further processing the emulsion particle obtained in the destruction step.

### (2-4-3) Detection step

According to one preferred embodiment of the present technology, detection or analysis of a component contained in the particle to be collected or reaction between the component contained in the particle to be collected and another component can be performed after the collection step.

In order to perform the detection or reaction, for example, an emulsion particle formed according to the present technology can be merged with another emulsion particle. Then, after the merging, the detection or analysis of the component contained in the particle to be collected or the reaction between the component and another component can be performed in the merged emulsion particle.

The merging makes it possible to perform, for example, Cellular Indexing of Transcriptomes and Epitopes by Sequencing (also called CITE-seq). For example, there is a case where the particle to be collected is a cell bound to an antibody to which an oligo barcode having a poly-A sequence is bound. An emulsion particle containing the cell is formed in the collection step. Then, after that, the emulsion particle is merged with an emulsion particle containing a bead or gel having a barcode sequence. The merging makes it possible to detect a cell surface protein or intracellular mRNA of the cell.

Further, the emulsified cell or cell aggregation (e.g., spheroid, organoid, or the like) may react with a chemical agent. This makes it possible to detect or analyze a response of the cell or cell aggregation to the chemical agent.

Further, a biomolecule may be detected in the detection. The biomolecule can be detected by, for example, wash free assay. In the wash free assay, for example, the LOCI, FRET, BRET, or FlimPIA method can be used.

### (2-4-4) Synthesis step

According to one preferred embodiment of the present technology, a chemical substance can be synthesized by the particle to be collected after the collection step. For example, the synthesis may be performed in the emulsion particle formed in the collection step. For example, an in vitro antibody can be prepared by injecting a cell-free expression reagent into the emulsion particle. Examples of the cell-free expression reagent include linear DNA and E. coli S30 Extract system for linear DNA (promega). Further, for example, protein synthesis may be performed by a cell-free protein synthesis system in the emulsion particle.

### (2-5) Fine particle sorting mechanism and fine particle

The fine particle sorting mechanism used in a method of sorting fine particles according to the present technology may be a structure or device having the channel structure described above, for example, may be a chip having a microchannel, in particular, a microchip for sorting fine particles.

In the present technology, the word "micro" means that at least a part of a channel included in the microchip for sorting fine particles has dimensions of µm order, in particular, a cross-sectional dimension of µm order. That is, in the present technology, the word "microchip" refers to a chip having a channel of µm order, and, in particular, a chip having a channel having a cross-sectional dimension of µm order. For example, a chip having a particle sorting portion formed by a channel having a cross-sectional dimension of µm order can be referred to as a microchip according to the present technology. For example, in the particle sorting portion 157, the main channel 155 can have, for example, a rectangular cross section, and a width of the main channel 155 in the particle sorting portion 157 can be, for example, 100 µm to 500 µm, and, in particular, 100 µm to 300 µm. The width of the bifurcating channels bifurcating from the main channel 155 may be smaller than the width of the main channel 155. The connection channel 170 can have, for example, a circular cross section, and the diameter of the connection channel 170 at the connection portion between the connection channel 170 and the main channel 155 can be, for example, 10 µm to 60 µm, and, in particular, 20 µm to 50 µm. Those dimensions regarding the channels may be appropriately changed in accordance with the size of the fine particle, in particular, the size of the particle to be collected.

The microchip 150 for sorting fine particles can be manufactured by a method known in the art. For example, the microchip 150 for sorting biological particles can be manufactured by laminating two or more substrates in which predetermined channels are formed. The channels may be formed in, for example, all of the two or more substrates (in particular, two substrates) or may be formed only on a part of the two or more substrates (in particular, one of two substrates). In order to easily adjust positions when the substrates are bonded, the channels are preferably formed only on one substrate.

The microchip 150 for sorting fine particles can be made from a material known in the art. Examples thereof include polycarbonate, cycloolefin polymers, polypropylene, polydimethylsiloxane (PDMS), polymethylmethacrylate (PMMA), polyethylene, polystyrene, glass, and silicon, but are not limited thereto. In particular, polymer materials such as, for example, polycarbonate, cycloolefin polymer, and polypropylene are particularly preferable because those polymer materials are excellent in processability and the microchip can be inexpensively manufactured by using a molding apparatus.

The microchip 150 for sorting fine particles is preferably transparent. For example, at least a part of the microchip 150 for sorting fine particles through which light (laser light and scattered light) passes can be transparent, and, for example, the detection region can be transparent. The entire microchip 150 for sorting fine particles may be transparent.

Note that, although the embodiment in which the above-described channel group is formed in the disposable microchip 150 for sorting fine particles has been described above, the channel group may not be formed in the microchip 150 in the present technology. For example, the above-described channel group may be formed in, for example, a plastic, glass, or other material substrate. Further, the above-described channel group may have a two-dimensional or three-dimensional structure.

In the present technology, the fine particle may be a particle having dimensions in which the particle can flow through the channel in the fine particle sorting mechanism (e.g., the microchip for sorting fine particles). In the present technology, the fine particle may be appropriately selected by a person skilled in the art. In the present technology, the fine particle can encompass a biological fine particle such as a cell, cell aggregation, microorganism, and liposome and a synthetic fine particle such as a gel particle, bead, latex particle, polymer particle, and industrial particle.

The biological fine particle (also referred to as biological particle) can encompass chromosomes, liposomes, mitochondria, organelles, and the like forming various cells. The cell can encompass an animal cell (e.g., hematopoietic cell) and plant cell. The cell can particularly be a hematopoietic cell or tissue cell. The hematopoietic cell may be, for example, a floating cell such as a T cell or B cell. The tissue cell may be, for example, an adherent cultured cell or adherent cell separated from a tissue. The cell aggregation can encompass, for example, a spheroid, organoid, and the like. The microorganism can encompass bacteria such as Escherichia coli, viruses such as a tobacco mosaic virus, and fungi such as a yeast. Further, the biological fine particle can also encompass biological polymers such as nucleic acids, proteins, and complexes thereof. Those biological polymers may, for example, be extracted from a cell or may be contained in a blood sample or other liquid samples.

The synthetic fine particle can be, for example, a fine particle made from an organic or inorganic polymer material, a metal, or the like. The organic polymer material can encompass polystyrene, styrenedivinylbenzene, polymethylmethacrylate, and the like. The inorganic polymer material can encompass glass, silica, magnetic materials, and the like. The metal can encompass colloidal gold, aluminum, and the like. The synthetic fine particle may be, for example, a gel particle or bead, and, more particularly, a gel particle or bead to which one or a combination of two or more selected from oligonucleotides, peptides, proteins, and enzymes is bound.

The fine particle may have a spherical, substantially spherical, or non-spherical shape. The size and mass of the fine particle can be appropriately selected by a person skilled in the art in accordance with the size of the channel of the microchip. Meanwhile, the size of the channel of the microchip can also be appropriately selected in accordance with the size and mass of the fine particle. In the present technology, for example, a chemical or biological label such as a fluorescent dye or fluorescent protein can be attached to the fine particle as necessary. The label makes it easier to detect the fine particle. The label to be attached can be appropriately selected by a person skilled in the art. The label can be bound to a molecule that specifically reacts with the fine particle (e.g., an antibody, aptamer, DNA, RNA, or the like).

According to one embodiment of the present technology, the fine particle can be a biological particle, in particular, a cell.

In the present technology, the fine particle collected into the collection channel may be subjected to further fine particle sorting processing. The further fine particle sorting processing may be performed by using, for example, the microchip 150 for sorting fine particles described above or another microchip for sorting fine particles.

For example, any one or a combination of two or more of the steps described in the above sections (2-1) to (2-4) may be performed in the further fine particle sorting processing. For example, in a case where the determination step described in the above section (2-2) is performed in the further fine particle sorting processing, in order to collect the emulsion particle containing the fine particle, the determination may be performed on the fine particle, the emulsion particle, or both the fine particle and emulsion particle in the determination step. As described above, also in the further fine particle sorting processing, whether or not the fine particle or emulsion particle is the particle to be collected may be determined on the basis of information obtained from the fine particle and/or emulsion particle.

Further, in the further fine particle sorting processing, an emulsion containing the emulsion particle containing the fine particle may be subjected to the sorting processing.

### (3) Other examples of channel structure

### (3-1) Channel structure in which main channel and waste channel are linearly arranged

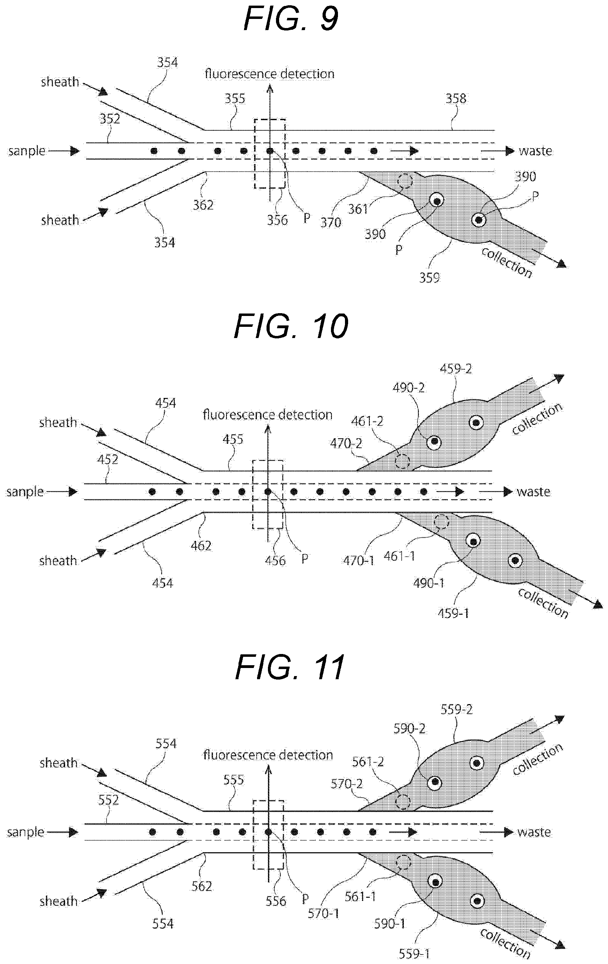

In one embodiment of the present technology, the connection channel and the collection channel may not be linearly arranged with the main channel. An example of the microchip for sorting fine particles in this embodiment will be described below with reference to Fig. 9.

Fig. 9 illustrates a schematic diagram of the detection region and a region including the particle sorting portion in the microchip for sorting fine particles according to the present technology. A microchip 350 for sorting fine particles illustrated in Fig. 9, as well as the microchip 150 for sorting fine particles described with reference to Fig. 1, has a channel structure in which a sample fluid channel 352 and sheath fluid channels 354 join at a joining portion 362 to form a main channel 355 and a detection region 356 is provided in the main channel 355. A laminar flow in which the sample fluid containing the fine particles P is surrounded by the sheath fluid flows through the main channel 355.

The microchip 350 for sorting fine particles includes not only the main channel 355 but also a collection channel 359 into which particles to be collected are collected, a connection channel 370 that connects the main channel 355 and the collection channel 359, and a liquid supply channel 361 connected to the connection channel 370 so as to supply a liquid.

The main channel 355 further includes a waste channel 358 through which fine particles that are not the particles to be collected flow. In the microchip 150 for sorting fine particles described with reference to Fig. 1, the two waste channels 158 bifurcate from the main channel 155 and are linearly arranged with the connection channel 170, the collection channel 159, and the main channel 155. Meanwhile, the microchip 350 for sorting fine particles in Fig. 9 has a channel structure in which the main channel 355 and the waste channel 358 are linearly arranged and the connection channel 370 and the collection channel 359 bifurcate from the main channel 355.

As described above, in the microchip for sorting fine particles used in the method of collecting fine particles according to the present technology, the connection channel and the collection channel may not be linearly arranged with the main channel. For example, the microchip may have a channel structure in which the main channel and the waste channel are linearly arranged and the connection channel and the collection channel bifurcate from the main channel.

### (3-2) Channel structure having plurality of collection channels

In another embodiment of the present technology, the microchip for sorting fine particles may have one or two or more channel structures each including a main channel through which the fine particles flow, a collection channel into which particles to be collected are collected among the fine particles, a connection channel that connects the main channel and the collection channel, and a liquid supply channel connected to the connection channel so as to supply a liquid. The microchip for sorting fine particles described above with reference to Fig. 1 has the one channel structure. Meanwhile, as described below with reference to Figs. 10 and 11, the microchip for sorting fine particles used in the method of collecting fine particles according to the present technology may have the two or more channel structures.

Fig. 10 illustrates a schematic diagram of the detection region and the region including the particle sorting portion in the microchip for sorting fine particles according to the present technology. A microchip 450 for sorting fine particles illustrated in Fig. 10, as well as the microchip 150 for sorting fine particles described with reference to Fig. 1, has a channel structure in which a sample fluid channel 452 and sheath fluid channels 454 join at a joining portion 462 to form a main channel 455 and a detection region 456 is provided in the main channel 455. A laminar flow in which the sample fluid containing the fine particles P is surrounded by the sheath fluid flows through the main channel 455.

The microchip 450 for sorting fine particles includes not only the main channel 455 but also a collection channel 459-1 into which particles to be collected are collected, a connection channel 470-1 that connects the main channel 455 and the collection channel 459-1, and a liquid supply channel 461-1 connected to the connection channel 470-1 so as to supply a liquid. The microchip 450 for sorting fine particles further includes a collection channel 459-2 into which particles to be collected are collected, a connection channel 470-2 that connects the main channel 455 and the collection channel 459-2, and a liquid supply channel 461-2 connected to the connection channel 470-2 so as to supply a liquid. That is, the microchip 450 for sorting fine particles has the two channel structures. In the microchip 450 for sorting fine particles, the main channel 455 is shared by those two channel structures. Further, the connection channel 470-1 is connected to the main channel 455 downstream of the connection channel 470-2.

The microchip 450 for sorting fine particles can form, for example, two emulsions containing different particles to be collected.

In this case, whether the fine particles are, for example, either of two kinds of particles A and B to be collected or neither of the two kinds of particles to be collected is determined in the determination step. A criterion regarding whether the fine particles belong to the particle A or B to be collected may be appropriately selected by a user.

In the collection step, in a case where the fine particles are the particles A to be collected, the fine particles are collected into the collection channel 459-1 through the connection channel 470-1. The fine particles that are the particles A to be collected are collected into the second liquid in the collection channel 459-1 while being contained in the first liquid.

In the collection step, in a case where the fine particles are the particles B to be collected, the fine particles are collected into the collection channel 459-2 through the connection channel 470-2. The fine particles that are the particles B to be collected are collected into the second liquid in the collection channel 459-2 while being contained in the first liquid.

In the collection step, in a case where the fine particles are neither of the particles A and B to be collected, the fine particles flow into the waste channel 458.

As described above, an emulsion containing the particles A to be collected and an emulsion containing the particles B to be collected are formed.

Fig. 11 illustrates a schematic view of the detection region and the region including the particle sorting portion in the microchip for sorting fine particles according to the present technology. A microchip 550 for sorting fine particles illustrated in Fig. 11 is the same as the microchip 450 for sorting fine particles described with reference to Fig. 10, except that two connection channels 570-1 and 570-2 are connected at the same position of a main channel 555. That is, the microchip 550 for sorting fine particles has the two channel structures, and the main channel 555 is shared by those two channel structures.

The microchip 550 for sorting fine particles can also form two emulsions containing different particles to be collected.

Also in this case, whether the fine particles are, for example, either of two kinds of particles A and B to be collected or neither of the two kinds of particles to be collected is determined in the determination step. A criterion regarding whether the fine particles belong to the particle A or B to be collected may be appropriately selected by a user.

In the collection step, in a case where the fine particles are the particles A to be collected, the fine particles are collected into the collection channel 559-1 through the connection channel 570-1. The fine particles that are the particles A to be collected are collected into the second liquid in the collection channel 559-1 while being contained in the first liquid.

In the collection step, in a case where the fine particles are the particles B to be collected, the fine particles are collected into the collection channel 559-2 through the connection channel 570-2. The fine particles that are the particles B to be collected are collected into the second liquid in the collection channel 559-2 while being contained in the first liquid.

In the collection step, in a case where the fine particles are neither of the particles A and B to be collected, the fine particles flow into the waste channel 558.

As described above, an emulsion containing the particles A to be collected and an emulsion containing the particles B to be collected are formed.

### 2. Second embodiment (microchip for sorting fine particles)

The present technology also provides a microchip for sorting fine particles having a channel structure including a main channel through which the fine particles flow, a collection channel into which particles to be collected are collected from among the fine particles, a connection channel that connects the main channel and the collection channel, and a liquid supply channel connected to the connection channel so as to supply a liquid. The main channel in the microchip has a determination region used for determining whether or not the fine particles flowing while being contained in a first liquid are the particles to be collected, and the fine particles determined as the particles to be collected are collected into a second liquid that is immiscible with the first liquid in the collection channel while being contained in the first liquid. The microchip for sorting fine particles is the same as the microchip for sorting fine particles described in the above section 1., and the description thereof also applies to the present embodiment.

### 3. Third embodiment (device for collecting fine particles)

The present technology also provides a device for collecting fine particles including: a microchip for sorting the fine particles having a channel structure including a main channel through which the fine particles flow, a collection channel into which particles to be collected are collected from among the fine particles, a connection channel that connects the main channel and the collection channel, and a liquid supply channel connected to the connection channel so as to supply a liquid; a first liquid supply unit that supplies a first liquid containing the fine particles to the main channel; a second liquid supply unit that supplies a second liquid that is immiscible with the first liquid to the liquid supply channel; and a determination unit that determines whether or not the fine particles flowing through the main channel are the particles to be collected. The device for collecting fine particles is the same as the device for collecting fine particles described in the above section 1., and the description thereof also applies to the present embodiment.

The microchip for sorting fine particles is also the same as the microchip for sorting fine particles described in the above section 1., and the description thereof also applies to the present embodiment.

The first liquid supply unit may be, for example, at least one component for forming the laminar flow described in the above section (2) in 1. and can have, for example, the sample fluid channel 152 and the sheath fluid channels 154. Further, the first liquid supply unit can also include: channels (e.g., tubes) connected to the sample fluid inlet 151 and the sheath fluid inlet 153, respectively, so as to introduce the sample fluid and the sheath fluid into those inlets; and a sample-fluid-containing container and a sheath-fluid-containing container connected to the channels.

The second liquid supply unit may be, for example, at least one component for supplying the second liquid to the liquid supply channels 161 described in the above section (2) in 1., and can include, for example, a container for containing the second liquid to be supplied to the liquid supply channels 161 and a channel that connects the container and the liquid supply channels (e.g., a tube that connects the chip and the container, or the like).

The determination unit is the same as the determination unit described in the above section 1., and the description thereof also applies to the present embodiment.

Further, the microchip for sorting fine particles may be detachable from the device for collecting fine particles. Because the microchip for sorting fine particles is detachable from the device, it is possible to use a new microchip for sorting fine particles for each sample. This makes it possible to prevent contamination of samples.

### 4. Fourth embodiment (method of producing emulsion)

The present technology also provides a method of producing an emulsion, the method including the flow step, the determination step, and the collection step described in the above section "1. First embodiment (method of collecting fine particles)". The production method may include other steps described in the above section "(2-4) Other steps". The description in the above section "1. First embodiment (method of collecting fine particles)" also applies to the method of producing an emulsion according to the present technology.

By the method of producing an emulsion according to the present technology, it is possible to produce an emulsion containing emulsion particles each containing one fine particle (in particular, a particle to be collected) at a high content ratio. For example, according to the production method of the present technology, it is possible to produce an emulsion in which the ratio of the number of emulsion particles each containing one fine particle to the total number of emulsion particles is, for example, 70% or more, preferably 80% or more, more preferably 90% or more, and further preferably 95% or more. In single cell analysis using an emulsion, it is important to increase a content ratio of emulsions each containing one cell, and, in a case where there are a small number of cells to be analyzed, it is particularly important to increase the content ratio. The production method of the present technology is extremely useful for the single cell analysis because it is possible to produce an emulsion in which the ratio of the number of emulsion particles each containing one fine particle is high.

### 5. Fifth embodiment (emulsion)

The present technology also provides an emulsion containing emulsion particles containing fine particles, in which the ratio of the number of emulsion particles each containing one fine particle to the total number of emulsion particles is 70% or more. The ratio is preferably 80% or more, more preferably 90% or more, and still more preferably 95% or more. Because the emulsion of the present technology contains emulsion particles each containing one fine particle at such a high content ratio, the emulsion is suitable for, for example, single cell analysis.

The emulsion particles each containing one fine particle can be formed as described in the above section "1. First embodiment (method of collecting fine particles)". Further, the emulsion can be produced as described in, for example, the above section "1. First embodiment (method of collecting fine particles)" or "4. Fourth embodiment (method of producing emulsion)".

The emulsion may be, for example, an emulsion containing the second liquid as the dispersion medium and the first liquid as the dispersoid. The description in the above section "1. First embodiment (method of collecting fine particles)" applies to those liquids. The description in the above section "1. First embodiment (method of collecting fine particles)" also applies to the fine particle contained in the emulsion particle, and the fine particle may be, for example, a cell, cell aggregation, synthetic particle, or the like.

Note that the present technology can also have the following configurations.
[1] A method of collecting fine particles, in which
   in a fine particle sorting mechanism having a channel structure including
   a main channel through which the fine particles flow,
   a collection channel into which particles to be collected are collected from among the fine particles,
   a connection channel that connects the main channel and the collection channel, and
   a liquid supply channel connected to the connection channel so as to supply a liquid,
   the method includes:
      a flow step of causing a first liquid containing the fine particles to flow through the main channel;
      a determination step of determining whether or not the fine particles flowing through the main channel are the particles to be collected; and
      a collection step of collecting the particles to be collected into the collection channel, and
      in the collection step, the particles to be collected are collected into a second liquid that is immiscible with the first liquid in the collection channel while being contained in the first liquid.
[2] The method of collecting the fine particles according to [1], in which an emulsion containing the second liquid as a dispersion medium and the first liquid as a dispersoid is formed in the collection channel by carrying out the method of collecting the fine particles.
[3] The method of collecting the fine particles according to [1] or [2], in which an emulsion is formed by carrying out the method of collecting the fine particles, and at least a part of droplets forming the emulsion contains one of the particles to be collected.
[4] The method of collecting the fine particles according to any one of [1] to [3], in which the first liquid is hydrophilic and the second liquid is hydrophobic.
[5] The method of collecting the fine particles according to any one of [1] to [4], in which a kinematic viscosity of the second liquid is 1/100 times to 100 times as much as the kinematic viscosity of the first liquid.
[6] The method of collecting the fine particles according to any one of [1] to [5], in which the flow step, the determination step, and the collection step are performed while the second liquid is being supplied from the liquid supply channel to the connection channel.
[7] The method of collecting the fine particles according to any one of [1] to [6], in which
   the main channel bifurcates into the connection channel and at least one waste channel through which a fine particle other than the particles to be collected flows, and
   the liquid supply channel supplies the liquid to the connection channel.
[8] The method of collecting the fine particles according to any one of [1] to [7], in which a valve for preventing the first liquid from entering the collection channel is provided in the connection channel.
[9] The method of collecting the fine particles according to any one of [1] to [8], in which in the flow step, the fine particles flow through the main channel substantially in a row toward the connection channel.
[10] The method of collecting the fine particles according to any one of [1] to [9], in which
   the fine particle sorting mechanism has a channel structure in which a sample channel through which a liquid containing the fine particles flows and a sheath channel through which a liquid containing no fine particle flows are connected to the main channel at a joining portion so that the fine particles flow substantially in a row through the main channel after the joining portion, and
   the channel structure forms a laminar flow containing the fine particles that flow in a row.
[11] The method of collecting the fine particles according to any one of [1] to [10], in which in the determination step, the fine particles flowing through the main channel are irradiated with light, and whether or not the fine particles are the particles to be collected is determined on the basis of light generated by the irradiation.
[12] The method of collecting the fine particles according to any one of [1] to [11], in which in the collection step, the particles to be collected are collected into the collection channel through the connection channel due to a pressure fluctuation in the collection channel.
[13] The method of collecting the fine particles according to any one of [1] to [12], in which the main channel, the connection channel, and the collection channel are linearly arranged.
[14] The method of collecting the fine particles according to any one of [1] to [13], in which the fine particles are cells or cell aggregation, and the first liquid is a culture solution of the fine particles.
[15] The method of collecting the fine particles according to any one of [1] to [14], in which the fine particles are cells, cell aggregation, or synthetic particles, and the fine particles are destroyed after the collection step.
[16] The method of collecting the fine particles according to any one of [1] to [13], in which the collected fine particles in the collection channel are subjected to further fine particle sorting processing.
[17] The method of collecting the fine particles according to any one of [1] to [6], in which the microchip for sorting fine particles has one or more channel structures.
[18] A microchip for sorting fine particles, in which the microchip has a channel structure including
   a main channel through which the fine particles flow,
   a collection channel into which particles to be collected are collected from among the fine particles,
   a connection channel that connects the main channel and the collection channel, and
   a liquid supply channel connected to the connection channel so as to supply a liquid,
   the main channel has a determination region used for determining whether or not the fine particles flowing while being contained in a first liquid are the particles to be collected, and
   the fine particles determined as the particles to be collected are collected into a second liquid that is immiscible with the first liquid in the collection channel while being contained in the first liquid.
[19] A device for collecting fine particles including:
   a microchip for sorting the fine particles having a channel structure including
   a main channel through which the fine particles flow,
   a collection channel into which particles to be collected are collected from among the fine particles,
   a connection channel that connects the main channel and the collection channel, and
   a liquid supply channel connected to the connection channel so as to supply a liquid;
   a first liquid supply unit that supplies a first liquid containing the fine particles to the main channel;
   a second liquid supply unit that supplies a second liquid that is immiscible with the first liquid to the liquid supply channel; and
   a determination unit that determines whether or not the fine particles flowing through the main channel are the particles to be collected.
[20] The device for collecting the fine particles according to [19], in which the microchip for sorting the fine particles is detachable from the device for collecting the fine particles.
[21] A method of producing an emulsion containing emulsion particles containing fine particles, in which
   in a fine particle sorting mechanism having a channel structure including
   a main channel through which the fine particles flow,
   a collection channel into which particles to be collected are collected from among the fine particles,
   a connection channel that connects the main channel and the collection channel, and
   a liquid supply channel connected to the connection channel so as to supply a liquid,
   the method includes:
      a flow step of causing a first liquid containing the fine particles to flow through the main channel;
      a determination step of determining whether or not the fine particles flowing through the main channel are the particles to be collected; and
      a collection step of collecting the particles to be collected into the collection channel, and
      in the collection step, the particles to be collected are collected into a second liquid that is immiscible with the first liquid in the collection channel while being contained in the first liquid.
[22] An emulsion containing emulsion particles containing fine particles, in which a ratio of the number of emulsion particles each containing one fine particle to the total number of emulsion particles is 70% or more.

### REFERENCE SIGNS LIST

- 100: Device for collecting fine particles
- 150: Microchip for sorting fine particles
- 155: Main channel
- 159: Collection channel
- 161: Liquid supply channel
- 170: Connection channel

## Claims

1. A method of collecting fine particles, wherein
in a fine particle sorting mechanism having a channel structure including
a main channel through which the fine particles flow,
a collection channel into which particles to be collected are collected from among the fine particles,
a connection channel that connects the main channel and the collection channel, and
a liquid supply channel connected to the connection channel so as to supply a liquid,
the method comprises:
a flow step of causing a first liquid containing the fine particles to flow through the main channel;
a determination step of determining whether or not the fine particles flowing through the main channel are the particles to be collected; and
a collection step of collecting the particles to be collected into the collection channel, and
in the collection step, the particles to be collected are collected into a second liquid that is immiscible with the first liquid in the collection channel while being contained in the first liquid.

2. The method of collecting the fine particles according to claim 1, wherein an emulsion containing the second liquid as a dispersion medium and the first liquid as a dispersoid is formed in the collection channel by carrying out the method of collecting the fine particles.

3. The method of collecting the fine particles according to claim 1, wherein an emulsion is formed by carrying out the method of collecting the fine particles, and at least a part of droplets forming the emulsion contains one of the particles to be collected.

4. The method of collecting the fine particles according to claim 1, wherein the first liquid is hydrophilic and the second liquid is hydrophobic.

5. The method of collecting the fine particles according to claim 1, wherein a kinematic viscosity of the second liquid is 1/100 times to 100 times as much as the kinematic viscosity of the first liquid.

6. The method of collecting the fine particles according to claim 1, wherein the flow step, the determination step, and the collection step are performed while the second liquid is being supplied from the liquid supply channel to the connection channel.

7. The method of collecting the fine particles according to claim 1, wherein
the main channel bifurcates into the connection channel and at least one waste channel through which a fine particle other than the particles to be collected flows, and
the liquid supply channel supplies the liquid to the connection channel.

8. The method of collecting the fine particles according to claim 1, wherein a valve for preventing the first liquid from entering the collection channel is provided in the connection channel.

9. The method of collecting the fine particles according to claim 1, wherein in the flow step, the fine particles flow through the main channel substantially in a row toward the connection channel.

10. The method of collecting the fine particles according to claim 1, wherein
the fine particle sorting mechanism has a channel structure in which a sample channel through which a liquid containing the fine particles flows and a sheath channel through which a liquid containing no fine particle flows are connected to the main channel at a joining portion so that the fine particles flow substantially in a row through the main channel after the joining portion, and
the channel structure forms a laminar flow containing the fine particles that flow substantially in a row.

11. The method of collecting the fine particles according to claim 1, wherein in the determination step, the fine particles flowing through the main channel are irradiated with light, and whether or not the fine particles are the particles to be collected is determined on a basis of light generated by the irradiation.

12. The method of collecting the fine particles according to claim 1, wherein in the collection step, the particles to be collected are collected into the collection channel through the connection channel due to a pressure fluctuation in the collection channel.

13. The method of collecting the fine particles according to claim 1, wherein the main channel, the connection channel, and the collection channel are linearly arranged.

14. The method of collecting the fine particles according to claim 1, wherein the fine particles are cells or cell aggregation, and the first liquid is a culture solution of the fine particles.

15. The method of collecting the fine particles according to claim 1, wherein the fine particles are cells, cell aggregation, or synthetic particles, and the fine particles are destroyed after the collection step.

16. The method of collecting the fine particles according to claim 1, wherein the collected fine particles in the collection channel are subjected to further fine particle sorting processing.

17. The method of collecting the fine particles according to claim 1, wherein the fine particle sorting mechanism has one or more channel structures.

18. A microchip for sorting fine particles, wherein the microchip has a channel structure including
a main channel through which the fine particles flow,
a collection channel into which particles to be collected are collected from among the fine particles,
a connection channel that connects the main channel and the collection channel, and
a liquid supply channel connected to the connection channel so as to supply a liquid,
the main channel has a determination region used for determining whether or not the fine particles flowing while being contained in a first liquid are the particles to be collected, and
the fine particles determined as the particles to be collected are collected into a second liquid that is immiscible with the first liquid in the collection channel while being contained in the first liquid.

19. A device for collecting fine particles, comprising:
a microchip for sorting the fine particles having a channel structure including
a main channel through which the fine particles flow,
a collection channel into which particles to be collected are collected from among the fine particles,
a connection channel that connects the main channel and the collection channel, and
a liquid supply channel connected to the connection channel so as to supply a liquid;
a first liquid supply unit that supplies a first liquid containing the fine particles to the main channel;
a second liquid supply unit that supplies a second liquid that is immiscible with the first liquid to the liquid supply channel; and
a determination unit that determines whether or not the fine particles flowing through the main channel are the particles to be collected.

20. The device for collecting the fine particles according to claim 19, wherein the microchip for sorting the fine particles is detachable from the device for collecting the fine particles.

21. A method of producing an emulsion containing emulsion particles containing fine particles, wherein
in a fine particle sorting mechanism having a channel structure including
a main channel through which the fine particles flow,
a collection channel into which particles to be collected are collected from among the fine particles,
a connection channel that connects the main channel and the collection channel, and
a liquid supply channel connected to the connection channel so as to supply a liquid,
the method comprises:
a flow step of causing a first liquid containing the fine particles to flow through the main channel;
a determination step of determining whether or not the fine particles flowing through the main channel are the particles to be collected; and
a collection step of collecting the particles to be collected into the collection channel, and
in the collection step, the particles to be collected are collected into a second liquid that is immiscible with the first liquid in the collection channel while being contained in the first liquid.

22. An emulsion containing emulsion particles containing fine particles, wherein a ratio of the number of emulsion particles each containing one fine particle to the total number of emulsion particles is 70% or more.
